# EUROPEAN PATENT SPECIFICATION

(11) **EP 1 542 701 B1**
(45) Date of publication and mention of the grant of the patent: **18.07.2012**
(21) Application number: 03749081.0
(22) Date of filing: 21.08.2003
(51) Int. Cl.: A61K 31/70, A61K 48/00, A61K 45/00, A01N 63/00, C12N 15/63, C12N 5/00, C12N 5/07, A61P 9/10

(54) **STROMAL CELL-DERIVED FACTOR-1 MEDIATES STEM CELL HOMING AND TISSUE REGENERATION IN ISCHEMIC CARDIOMYOPATHY**
AUS STROMAZELLEN STAMMENDER FAKTOR-1 VERMITTELT STAMMZELLEN-HOMING UND GEWEBEREGENERATION BEI ISCHûMISCHER KARDIOMYOPATHIE
ECOTAXIE DES CELLULES SOUCHES ET REGÉNÉRATION TISSULAIRE MÉDIÉES PAR SDF-1 DANS LA MYOCARDIOPATHIE ISCHÉMIQUE

(30) Priority: 22.08.2002 US 405274 P; 30.04.2003 US 426712
(43) Date of publication of application: 22.06.2005
(60) Divisional application: 12164577.4
(73) Proprietor: THE CLEVELAND CLINIC FOUNDATION, Cleveland, OH 44195 (US)
(72) Inventor: PENN, Marc, S., Shaker Heights, OH 44122 (US); ASKARI, Arman, T., Orange, Ohio 44022 (US); KIEDROWSKI, Matthew, Lorain, OH 44053 (US)
(74) Representative: Horner, Martin Grenville
(86) International application number: PCT/US2003/026013
(87) International publication number: WO 2004/017978

(56) References cited:
- WO-A-02/09650
- WO-A1-99/45775
- US-A1- 2002 061 587
- SUZUKI K ET AL: "Cell transplantation for the treatment of acute myocardial infarction using vascular endothelial growth factor-expressing skeletal myoblasts" CIRCULATION, LIPPINCOTT WILLIAMS & WILKINS, US, vol. 104, no. SUPPL. 1, 18 September 2001 (2001-09-18), pages I-207, XP002974269 ISSN: 0009-7322
- ORLIC D ET AL: "MOBILIZED BONE MARROW CELLS REPAIR THE INFARCTED HEART, IMPROVING FUNCTION AND SURVIVAL" PROCEEDINGS OF THE NATIONAL ACADEMY OF SCIENCES OF USA, NATIONAL ACADEMY OF SCIENCE, WASHINGTON, DC, US, vol. 98, no. 18, 28 August 2001 (2001-08-28), pages 10344-10349, XP002950780 ISSN: 0027-8424
- ASKARI A T ET AL: "Stromal cell-derived factor-1 mediates stem cell homing in ischemic cardiomyopathy" CIRCULATION, LIPPINCOTT WILLIAMS & WILKINS, US, vol. 106, no. 19 suppl. 2, 5 November 2002 (2002-11-05), page 891, XP008112884 ISSN: 0009-7322
- ORLIC D ET AL: "Bone marrow cells regenerate infarcted myocardium" NATURE, NATURE PUBLISHING GROUP, LONDON, UK, vol. 410, 5 April 2001 (2001-04-05), pages 701-705, XP002965971 ISSN: 0028-0836
- ASKARI ARMAN T ET AL: "Effect of stromal-cell-derived factor 1 on stem-cell homing and tissue regeneration in ischaemic cardiomyopathy." LANCET (NORTH AMERICAN EDITION), vol. 362, no. 9385, 30 August 2003 (2003-08-30), pages 697-703, XP002549498 ISSN: 0099-5355
- PETIT I ET AL: "G-CSF induces stem cell mobilization by decreasing bone marrow SDF-1 and up-regulating CXCR4" NATURE IMMUNOLOGY 2002 US, vol. 3, no. 7, 17 June 2002 (2002-06-17), pages 687-694, XP002549499 ISSN: 1529-2908
- J MOL CELL CARDIOL, vol. 42, 2007, pages 792-803,
- THE LANCET, vol. 362, 30 August 2003 (2003-08-30), pages 697-703,

## Description

### Field of the Invention

The present invention relates to compositions for use in tissue regeneration in ischemic cardiomyopathy and particularly relates to compositions for use in treating ischemic cardiomyopathy at a time remote (i.e., weeks) from myocardial infarction.

### Background of the Invention

Acute myocardial infarction (MI) remains the leading cause of morbidity and mortality in western society. Despite recent therapeutic advances predominantly targeted at restoring antegrade perfusion in the infarct-related artery, a "ceiling" of benefit appears to exist. Topol, E.J. Lancet 357, 1905-1914 (2001). A substantial proportion of patients who experience an acute myocardial infarction (MI) ultimately develop congestive heart failure (CHF) largely as a result of left ventricular (LV) remodeling, a process involving myocardial thinning, dilation, decreased function, ultimately leading to death. Robbins, M.A. & O'Connell, J.B., pp. 3-13 (Lippincott-Raven, Philadelphia,1998). Pfeffer, J.M., Pfeffer, M.A., Fletcher, P.J. & Braunwald, E. Am. J. Physiol 260, H1406-H1414 (1991). Pfeffer, M.A. & Braunwald, E. Circulation 81, 1161-1172 (1990).

One method to treat this process following myocardial infarction involves cell therapy. Penn, M.S. et al. Prog. Cardiovasc. Dis. 45, 21-32 (2002) . Transplantation has focused on using a variety of cell types including differentiated cells, such as skeletal myoblasts, cardiac myocytes, smooth muscle cells, and fibroblasts, or bone marrow derived cells. Koh, G.Y., Klug, M.G., Soonpaa, M.H. & Field, L.J. J. Clin. Invest 92, 1548-1554 (1993). Taylor, D.A. et al. Nat. Med. 4, 929-933 (1998). Jain, M. et al. Circulation 103, 1920-1927 (2001). Li, R.K. et al. Ann. Thorac. Surg. 62, 654-660 (1996). Etzion, S. et al. J. Mol. Cell Cardiol. 33, 1321-1330 (2001). Li, R.K., Jia, Z.Q., Weisel, R.D., Merante, F. & Mickle, D.A. J. Mol. Cell Cardiol. 31, 513-522 (1999). Yoo, K.J. et al. Yonsei Med. J. 43, 296-303 (2002). Sakai, T. et al. Ann. Thorac. Surg. 68, 2074-2080 (1999). Sakai, T. et al. J. Thorac. Cardiovasc. Surg. 118, 715-724 (1999). Orlic, D. et al. Nature 410, 701-705 (2001). Tomita, S. et al. J. Thorac. Cardiovasc. Surg. 123, 1132-1140 (2002).

A growing body of literature suggests that stem cell mobilization to the heart and differentiation into cardiac myocytes is a naturally occurring process. Jackson, K.A. et al. J. Clin. Invest 107, 1395-1402 (2001). Quaini, F. et al. N. Engl. J. Med. 346, 5-15 (2002). This process occurs at a rate insufficient to result in meaningful recovery of left ventricular function following myocardial infarction. *Id.* Recently, studies have demonstrated the possibility of regenerating damaged myocardium either through the direct injection of stem cells into the blood stream, or via chemical mobilization of stem cells from the bone marrow prior to the myocardial infarction. These studies have demonstrated the ability of stem cells to home to the infarct zone in the peri-infarct period, as well as for these cells to then differentiate into cardiac myocytes. Kocher, A.A. et al. Nat. Med. 7, 430-436 (2001). Orlic, D. et al. Proc. Natl. Acad. Sci. U. S. A 98, 10344-10349 (2001). Peled, A. et al. Blood 95, 3289-3296 (2000). Yong, K. et al. Br. J. Haematol. 107, 441-449 (1999). To date, all the studies have focused on the ability of stem cells to regenerate myocardium within 48 hours after myocardial infarction.

### Summary of the Invention

The present invention is the following :
1. Use of a cell transfected with an expression vector encoding SDF-I protein to express SDF-1 protein, for the manufacture of a medicament for treating infarcted myocardial tissue in a subject.
2. A cell transfected with an expression vector encoding SDF-1 protein to express SDF-1 protein for use in treating infarcted myocardial tissue in a subject.
3. The use of 1 or cell for use of 2, comprising cardiofibroblasts genetically modified to express SDF-1 protein.
4. The use or cell for use of 3, the cardiofibroblasts being genetically modified with an expression vector, the expression vector including a nucleic acid encoding for SDF-1 protein.
5. The use or cell for use of 3, the cardiofibroblasts being cultured *ex-vivo.*
6. The use or cell for use of 5, the cardiofibroblasts being introduced into infarcted myocardial tissue by transplanting the cardiofibroblasts into the infarcted myocardial tissue.

### Brief Description of the Drawings

Features of the present disclosure will become apparent to those skilled in the art to which the present disclosure relates from reading the following description of the disclosure made with reference to the accompanying drawings in which:
Figs. 1(a and b) are graphs showing, respectively, the number BrdU-positive cells within infarct zone (a) and shortening fraction (b) 4 weeks following administration of saline or G-CSF (12 weeks following LAD ligation) .
Figs. 2(a and b) are graphs showing the effect of skeletal myoblast (SKMB) transplantation on BrdU+ cell counts within the infarct zone 4 weeks following cell transplantation (12 weeks following LAD ligation).
Figs. 3(a and b) are photographs showing (a) bone marrow stained for BrdU and (b) untreated myocardium after 5 days of BrdU administration.
Fig. 3c is a graph showing the increased BrdU+ cells within the infarct zone assessed with the therapy in accordance with the present invention.
Fig. 4 is a photograph showing RT-PCR revealing stromal derived factor-1 (SDF-1) expression as a function of time following myocardial infarction.
Figs. 5(a and b) are graphs showing the number of (a) BrdU+ cells and (b) CD117+ cells within the infarct zone 4 weeks following transplantation of cardiac fibroblasts stably transfected with or without SDF-1 expression vector with or without G-CSF administration for 5 days following cardiac fibroblast transplantation.
Fig. 5c is a photograph from a SDF-1/G-CSF treated animal stained CD117+.
Figs. 6(a and b) are photographs showing the immunohistochemistry of the infarct zone revealing both BrdU+ cells and cardiac myosin-expressing cells 12 weeks following LAD ligation with cell transplantation of (a) SKMB or (b) VEGF-expressing SKMB followed by stem cell mobilization using G-CSF.
Fig. 6c is a graph showing improvement in left ventricle function relative to cell therapy without VEGF over-expression.

### Description of the Embodiments

Unless otherwise defined, all technical terms used herein have the same meaning as commonly understood by one of ordinary skill in the art to which this invention belongs. Commonly understood definitions of molecular biology terms can be found in, for example, Rieger et al., Glossary of Genetics: Classical and Molecular, 5th edition, Springer-Verlag: New York, 1991; and Lewin, Genes V, Oxford University Press: New York, 1994.

Methods involving conventional molecular biology techniques are described herein. Such techniques are generally known in the art and are described in detail in methodology treatises, such as Molecular Cloning: A Laboratory Manual, 2nd ed., vol. 1-3, ed. Sambrook et al., Cold Spring Harbor Laboratory Press, Cold Spring Harbor, N.Y., 1989; and Current Protocols in Molecular Biology, ed. Ausubel et al., Greene Publishing and Wiley-Interscience, New York, 1992 (with periodic updates). Methods for chemical synthesis of nucleic acids are discussed, for example, in Beaucage and Carruthers, Tetra. Letts. 22:1859-1862, 1981, and Matteucci et al., J. Am. Chem. Soc. 103:3185, 1981. Chemical synthesis of nucleic acids can be performed, for example, on commercial automated oligonucleotide synthesizers. Immunological methods (e.g., preparation of antigen-specific antibodies, immunoprecipitation, and immunoblotting) are described, e.g., in Current Protocols in Immunology, ed. Coligan et al., John Wiley & Sons, New York, 1991; and Methods of Immunological Analysis, ed. Masseyeff et al., John Wiley & Sons, New York, 1992. Conventional methods of gene transfer and gene therapy can also be adapted for use in the present disclosure. See, e.g., Gene Therapy: Principles and Applications, ed. T. Blackenstein, Springer Verlag, 1999; Gene Therapy Protocols (Methods in Molecular Medicine), ed. P. D. Robbins, Humana Press, 1997; and Retro-vectors for Human Gene Therapy, ed. C. P. Hodgson, Springer Verlag, 1996.

The present disclosure relates to a method of myocardium regeneration in ischemic regions of the heart (or skeletal muscle in the case of peripheral vascular tissue). The method of the present disclosure can be used to treat ischemic cardiomyopathy at a time remote (i.e., weeks) from myocardial infarction.

The method includes mobilizing and directing migration of pluripotent stem cells to infarcted myocardium within a mammalian subject. Mammalian subjects can include any mammal, such as human beings, rats, mice, cats, dogs, goats, sheep, horses, monkeys, apes, rabbits, cattle, etc. The mammalian subject can be in any stage of development including adults, young animals, and neonates. Mammalian subjects can also include those in a fetal stage of development.

The infarcted myocardium can include the infarcted myocardial tissue, the myocardial tissue about the periphery of the infarcted myocardial tissue, and both the infarcted myocardial tissue and myocardial tissue about the periphery of the infarcted myocardial tissue.

At a time remote from myocardial infarction, a first number of pluripotent stem cells traffic the infarcted myocardium. This first number of stem cells can be increased so that a greater number of stem cells traffic the infarcted myocardium. By increasing the number of stem cells that traffic the infarcted myocardium, the infarcted myocardial tissue can be regenerated because there will be a greater number of pluripotent stem cells in the infarcted myocardium that can differentiate into cells, which can repopulate (i.e., engraft) and partially or wholly restore the normal function of the infarcted myocardium.

In accordance with one aspect of the present disclosure, invention pluripotent stem cells are induced to home to infarcted myocardium to regenerate infarcted myocardium. Pluripotent stem cells described in the invention are any cells that can be induced to differentiate into another cell type. One example includes hematopoietic stem cells that can differentiate into cardiomyocyte cells.

The pluripotent stem cells can be homed to the infarcted myocardium by increasing the concentration of SDF-1 protein within the infarcted myocardium from a first concentration to a second concentration. The first concentration of SDF-1 protein can be the concentration of SDF-1 protein typically found in an infarcted myocardium at a time remote (i.e., weeks) from the myocardial infarction. The second concentration of SDF-1 protein can be substantially greater than the first concentration of SDF-1 protein. The concentration of SDF-1 protein can be increased in the infarcted myocardium by up-regulating the expression of SDF-1 protein within the infarcted myocardium from the amount of SDF-1 protein typically expressed in the infarcted myocardium at a time remote from the myocardial infarction.

The method of the present disclosure includes a step of increasing the concentration (i.e., number) of stems cells in the peripheral blood from a first concentration to a second concentration substantially greater than the first concentration. The first concentration of stem cells can be the concentration of stem cells typically found in the peripheral blood at a time remote from the myocardial infarction. The concentration of stem cells in the peripheral blood can be increased while the concentration of SDF-1 protein in the infarcted myocardium is increased. The concentration of stem cells in the peripheral blood can be increased either before or after the SDF-1 protein expression is up-regulated in the infarcted myocardium.

The SDF-1 protein expressed by the infarcted myocardium induces the stem cells in the peripheral blood to the infarcted myocardium. The stems cells induced to the infarcted myocardium facilitate myocardial regeneration and provide a substantial increase in left ventricular function.

In accordance with another aspect of the present disclosure, the method can further include the step of increasing the concentration of vascular endothelial growth factor (VEGF) in the infarcted myocardium of the peripheral blood from a first concentration to a second concentration substantially greater than the first concentration. The first concentration of VEGF in the infarcted myocardium is the concentration of stem cells typically found in the infarcted myocardium at a time remote from the myocardial infarction. The concentration of VEGF can be increased in the infarcted myocardium by up-regulating the expression VEGF protein within the infarcted myocardium from the amount of VEGF typically expressed in the infarcted myocardium at time remote from the myocardial infarction.

The concentration of VEGF in the infarcted myocardium can be increased while the concentration of SDF-1 protein in infarcted myocardium is increased and the concentration of stem cell in the peripheral blood is increased. Increasing the concentration of vascular endothelial growth factor in the infarcted myocardium in combination with increasing the concentration of SDF-1 protein in the infarcted myocardium and increasing the concentration of stem cell in the peripheral blood increases vascular density within the infarcted myocardium compared to saline controls. Furthermore, this combination led to the repopulating of the infarcted myocardium with cardiac myosin expressing cells as well as an increase in left ventriclular function as measured by shortening fraction.

### SDF-1 protein

In accordance with one aspect of the present disclosure, the SDF-1 protein (or SDF-1 polypeptide) that is expressed in the infarcted myocardium can be an expression product of an SDF-1 gene. The amino acid sequence of a number or different mammalian SDF-1 proteins are known including, human, rat, mouse, and cat. The SDF-1 protein can have an amino acid sequence identical to one of the foregoing native mammalian SDF-1 proteins.

The SDF-1 protein of the present disclosure can also be a variant of mammalian SDF-1 protein, such as a fragment, analog and derivative of mammalian SDF-1 protein. Such variants include, for example, a polypeptide encoded by a naturally occurring allelic variant of native SDF-1 gene (i.e., a naturally occurring nucleic acid that encodes a naturally occurring mammalian SDF-1 protein), a polypeptide encoded by an alternative splice form of a native SDF-1 gene, a polypeptide encoded by a homolog of a native SDF-1 gene, and a polypeptide encoded by a non-naturally occurring variant of a native SDF-1 gene.

SDF-1 protein variants have a peptide sequence that differs from a native SDF-1 protein in one or more amino acids. The peptide sequence of such variants can feature a deletion, addition, or substitution of one or more amino acids of a SDF-1 protein. Amino acid insertions are preferably of about 1 to 4 contiguous amino acids, and deletions are preferably of about 1 to 10 contiguous amino acids. Variant SDF-1 proteins substantially maintain a native SDF-1 protein functional activity. Preferred SDF-1 protein variants can be made by expressing nucleic acid molecules within the invention that feature silent or conservative changes.

SDF-1 protein fragments corresponding to one or more particular motifs and/or domains or to arbitrary sizes, are within the scope of the present disclosure. Isolated peptidyl portions of SDF-1 proteins can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry. For example, a SDF-1 protein of the present disclosure may be arbitrarily divided into fragments of desired length with no overlap of the fragments, or preferably divided into overlapping fragments of a desired length. The fragments can be produced recombinantly and tested to identify those peptidyl fragments which can function as agonists of native SDF-1 protein.

Variants of SDF-1 protein can also include recombinant forms of the SDF-1 proteins. Recombinant polypeptides preferred by the present disclosure, in addition to a SDF-1 protein, are encoded by a nucleic acid that can have at least 85% sequence identity with the nucleic acid sequence of a gene encoding a mammalian SDF-1 protein.

SDF-1 protein variants can include agonistic forms of the protein that constitutively express the functional activities of a native SDF-1 protein. Other SDF-1 protein variants can include those that are resistant to proteolytic cleavage, as for example, due to mutations, which alter protease target sequences. Whether a change in the amino acid sequence of a peptide results in a variant having one or more functional activities of a native SDF-1 protein can be readily determined by testing the variant for a native SDF-1 protein functional activity.

### Nucleic Acids

Another aspect of the present disclosure relates to nucleic acid molecules that encode an SDF-1 protein and non-native nucleic acids that encode an SDF-1 protein. Such nucleic acid molecules may be in the form of RNA or in the form of DNA (e.g., cDNA, genomic DNA, and synthetic DNA). The DNA may be double-stranded or single-stranded, and if single-stranded may be the coding (sense) strand or non-coding (anti-sense) strand. The coding sequence which encodes an SDF-1 protein may be identical to a nucleotide sequence shown GenBank Accession No. AF189724, GenBank Accession No. AF209976, GenBank Accession No. L120029, and GenBank Accession No. NM022177. It may also be a different coding sequence which, as a result of the redundancy or degeneracy of the genetic code, encodes the same polypeptide as such polynucleotides.

Other nucleic acid molecules that encode SDF-1 within the disclosure are variants of a native SDF-1, such as those that encode fragments, analogs and derivatives of a native SDF-1 protein. Such variants may be, for example, a naturally occurring allelic variant of a native SDF-1 gene, a homolog of a native SDF-1 gene, or a non-naturally occurring variant of a native SDF-1 gene. These variants have a nucleotide sequence that differs from a native SDF-1 gene in one or more bases. For example, the nucleotide sequence of such variants can feature a deletion, addition, or substitution of one or more nucleotides of a native SDF-1 gene. Nucleic acid insertions are preferably of about 1 to 10 contiguous nucleotides, and deletions are preferably of about 1 to 10 contiguous nucleotides.

In other applications, variant SDF-1 proteins displaying substantial changes in structure can be generated by making nucleotide substitutions that cause less than conservative changes in the encoded polypeptide. Examples of such nucleotide substitutions are those that cause changes in (a) the structure of the polypeptide backbone; (b) the charge or hydrophobicity of the polypeptide; or (c) the bulk of an amino acid side chain. Nucleotide substitutions generally expected to produce the greatest changes in protein properties are those that cause non-conservative changes in codons. Examples of codon changes that are likely to cause major changes in protein structure are those that cause substitution of (a) a hydrophilic residue, e.g., serine or threonine, for (or by) a hydrophobic residue, e.g., leucine, isoleucine, phenylalanine, valine or alanine; (b) a cysteine or proline for (or by) any other residue; (c) a residue having an electropositive side chain, e.g., lysine, arginine, or histidine, for (or by) an electronegative residue, e.g., glutamine or aspartine; or (d) a residue having a bulky side chain, e.g., phenylalanine, for (or by) one not having a side chain, e.g., glycine.

Naturally occurring allelic variants of a native SDF-1 gene within the disclosure are nucleic acids isolated from mammalian tissue that have at least 75% sequence identity with a native SDF-1 gene, and encode polypeptides having structural similarity to a native SDF-1 protein. Homologs of a native SDF-1 gene within the disclosure are nucleic acids isolated from other species that have at least 75% sequence identity with the native gene, and encode polypeptides having structural similarity to a native SDF-1 protein. Public and/or proprietary nucleic acid databases can be searched to identify other nucleic acid molecules having a high percent (e.g., 70% or more) sequence identity to a native SDF-1 gene.

Non-naturally occurring SDF-1 gene variants are nucleic acids that do not occur in nature (e.g., are made by the hand of man), have at least 75% sequence identity with a native SDF-1 gene, and encode polypeptides having structural similarity to a native SDF-1 protein. Examples of non-naturally occurring SDF-1 gene variants are those that encode a fragment of a native SDF-1 protein, those that hybridize to a native SDF-1 gene or a complement of to a native SDF-1 gene under stringent conditions, those that share at least 65% sequence identity with a native SDF-1-gene or a complement of a native SDF-1 gene, and those that encode a SDF-1 fusion protein.

Nucleic acids encoding fragments of a native SDF-1 protein within the disclosure are those that encode, amino acid residues of a native SDF-1 protein. Shorter oligonucleotides that encode or hybridize with nucleic acids that encode fragments of a native SDF-1 protein can be used as probes, primers, or antisense molecules. Longer polynucleotides that encode or hybridize with nucleic acids that encode fragments of a native SDF-1 protein can also be used in various aspects of the disclosure. Nucleic acids encoding fragments of a native SDF-1 can be made by enzymatic digestion (e.g., using a restriction enzyme) or chemical degradation of the full length native SDF-1 gene or variants thereof.

Nucleic acids that hybridize under stringent conditions to one of the foregoing nucleic acids can also be used in the disclosure. For example, such nucleic acids can be those that hybridize to one of the foregoing nucleic acids under low stringency conditions, moderate stringency conditions, or high stringency conditions are within the disclosure made.

Nucleic acid molecules encoding a SDF-1 fusion protein may also be used in the disclosure. Such nucleic acids can be made by preparing a construct (e.g., an expression vector) that expresses a SDF-1 fusion protein when introduced into a suitable target cell. For example, such a construct can be made by ligating a first polynucleotide encoding a SDF-1 protein fused in frame with a second polynucleotide encoding another protein such that expression of the construct in a suitable expression system yields a fusion protein.

The oligonucleotides of the disclosure can be DNA or RNA or chimeric mixtures or derivatives or modified versions thereof, single-stranded or double-stranded. Such oligonucleotides can be modified at the base moiety, sugar moiety, or phosphate backbone, for example, to improve stability of the molecule, hybridization, etc. Oligonucleotides within the disclosure may additionally include other appended groups such as peptides (e.g., for targeting target cell receptors in vivo), or agents facilitating transport across the cell membrane, hybridization-triggered cleavage. To this end, the oligonucleotides may be conjugated to another molecule, e.g., a peptide, hybridization triggered cross-linking agent, transport agent, hybridization-triggered cleavage agent, etc.

### SDF-1 Expression

In accordance with another aspect of present disclosure, the expression of SDF-1 in the infarcted myocardium can be upregulated by transplanting biocompatible cells into the infarcted myocardial tissue. Transplantation of cells into the infarcted myocardium up-regulates the expression of SDF-1 protein in the infarcted myocardium. The up-regulation of SDF-1 protein in the infarcted myocardium was observed from about 1 hour after transplantation of cells into the myocardium to less than about 7 days after transplantation.

Examples of cell types that can be transplanted into the infarcted myocardium include cultured heart cells, skeletal myoblasts, fibroblasts, smooth muscle cells, and bone marrow derived cells. These cells are preferably harvested from the subject to be treated (i.e., autologous cells) and cultured prior to transplantation. Autologous cells are preferred in order to increase the biocompatibily of the cells upon transplantation and minimize the likelihood of rejection.

Preferred cells for transplantation into the infarcted myocardium are skeletal myoblasts. Myoblasts maintain the regenerative potential of skeletal muscle, during periods of stress, proliferate and differentiate into myotubes, eventually forming muscle fibers capable of contracting. Myoblasts implanted into myocardium undergo myotube formation, withdraw from cell cycle, and remain viable. Functional studies have shown an improvement in regional contractility and compliance after myoblast implantation into the myocardium.

Skeletal myoblasts can be readily harvested under the basal membrane of muscular fibers, cultured to scale up the cell line, and then transplanted into infarcted myocardium. For example, in a murine subject, skeletal myoblasts can be harvested from the hind limbs of the subject, cultured, and then transplanted into the infarcted myocardium of the subject.

The cultured cells can be transplanted in the infarcted myocardium by well-known cell transplant techniques. For example, a suspension of cultured cells can be injected using a tuberculin syringe into the infarcted myocardial tissue.

Alternatively, the expression of SDF-1 protein can be upregulated by introducing an agent into the target cells that increases expression of SDF-1 protein. The target cells can include cells within the infarcted myocardium or ex vivo cells, such as autologous skeletal myoblasts or fibroblasts, which have been harvested from the subject.

The agent can comprise natural or synthetic nucleic acids, according to present disclosure and described above, that are incorporated into recombinant nucleic acid constructs, typically DNA constructs, capable of introduction into and replication in the cell. Such a construct preferably includes a replication system and sequences that are capable of transcription and translation of a polypeptide-encoding sequence in a given target cell.

Other agents can also be introduced into the target cells to increase SDF-1 protein levels in the target tissue. For example, agents that increase the transcription of a gene encoding SDF-1 protein increase the translation of an mRNA encoding SDF-1 protein, and/or those that decrease the degradation of an mRNA encoding SDF-1 protein could be used to increase SDF-1 protein levels. Increasing the rate of transcription from a gene within a cell can be accomplished by introducing an exogenous promoter upstream of the gene encoding SDF-1 protein. Enhancer elements which facilitate expression of a heterologous gene may also be employed.

A preferred method of introducing the agent into a target cell involves using gene therapy. Gene therapy refers to gene transfer to express a therapeutic product from a cell in vivo or in vitro. Gene therapy in accordance with the present invention can be used to express SDF-1 protein from a target cell in vivo or in vitro.

One method of gene therapy uses a vector including a nucleotide encoding an SDF-1 protein. A "vector" (sometimes referred to as gene delivery or gene transfer "vehicle") refers to a macromolecule or complex of molecules comprising a polynucleotide to be delivered to a target cell, either in vitro or in vivo. The polynucleotide to be delivered may comprise a coding sequence of interest in gene therapy. Vectors include, for example, viral vectors (such as adenoviruses ('Ad'), adeno-associated viruses (AAV), and retroviruses), liposomes and other lipid-containing complexes, and other macromolecular complexes capable of mediating delivery of a polynucleotide to a target cell.

Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities.

Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (i.e. positive/negative) markers (see, e.g., Lupton, S., WO 92/08796, published May 29, 1992; and Lupton, S., WO 94/28143, published Dec. 8, 1994). Such marker genes can provide an added measure of control that can be advantageous in gene therapy contexts. A large variety of such vectors are known in the art and are generally available.

Vectors for use in the present disclosure include viral vectors, lipid based vectors and other vectors that are capable of delivering a nucleotide according to the present invention to the target cells. The vector can be a targeted vector, especially a targeted vector that preferentially binds to cardiomyocytes. Preferred viral vectors for use in the disclosure are those that exhibit low toxicity to a target cell and induce production of therapeutically useful quantities of SDF-1 protein in a tissue-specific manner.

Presently preferred viral vectors are derived from adenovirus (Ad) or adeno-associated virus (AAV). Both human and non-human viral vectors can be used but preferably the recombinant viral vector is replication-defective in humans. Where the vector is an adenovirus, it preferably comprises a polynucleotide having a promoter operably linked to a gene encoding the SDF-1 protein and is replication-defective in humans.

Adenovirus vectors are preferred for use in the present disclosure because they (1) are capable of highly efficient gene expression in target cells and (2) can accommodate a relatively large amount of heterologous (non-viral) DNA. A preferred form of recombinant adenovirus is a "gutless, "high-capacity", or "helper-dependent" adenovirus vector. Such a vector features, for example, (1) the deletion of all or most viral-coding sequences (those sequences encoding viral proteins), (2) the viral inverted terminal repeats (ITRs) which are sequences required for viral DNA replication, (3) up to 28-32 kb of "exogenous" or "heterologous" sequences (e.g., sequences encoding a SDF-1 protein), and (4) the viral DNA packaging sequence which is required for packaging of the viral genomes into infectious capsids. For specifically myocardial cells, preferred variants of such recombinant adenoviral vectors contain tissue-specific (e.g., cardiomyoctye) enhancers and promoters operably linked to a SDF-1 gene.

AAV-based vectors are advantageous because they exhibit high transduction efficiency of target cells and can integrate into the target genome in a site-specific manner. Use of recombinant AAV vectors is discussed in detail in Tal, J., J. Biomed. Sci. 7:279-291, 2000 and Monahan and Samulski, Gene Therapy 7:24-30, 2000. A preferred AAV vector comprises a pair of AAV inverted terminal repeats which flank at least one cassette containing a tissue (e.g., myocardium)--or cell (e.g., cardiomyocyte)--specific promoter operably linked to a SDF-1 nucleic acid. The DNA sequence of the AAV vector, including the ITRs, the promoter and SDF-1 gene may be integrated into the target genome.

Other viral vectors that can be use in accordance with the present disclosure include herpes simplex virus (HSV)-based vectors. HSV vectors deleted of one or more immediate early genes (IE) are advantageous because they are generally non-cytotoxic, persist in a state similar to latency in the target cell, and afford efficient target cell transduction. Recombinant HSV vectors can incorporate approximately 30 kb of heterologous nucleic acid. A preferred HSV vector is one that: (1) is engineered from HSV type I, (2) has its IE genes deleted, and (3) contains a tissue-specific (e.g., myocardium) promoter operably linked to a SDF-1 nucleic acid. HSV amplicon vectors may also be useful in various methods of the disclosure. Typically, HSV amplicon vectors are approximately 15 kb in length, and possess a viral origin of replication and packaging sequences.

Retroviruses such as C-type retroviruses and lentiviruses might also be used in the disclosure. For example, retroviral vectors may be based on murine leukemia virus (MLV). See, e.g., Hu and Pathak, Pharmacol. Rev. 52:493-511, 2000 and Fong et al., Crit. Rev. Ther. Drug Carrier Syst. 17:1-60, 2000. MLV-based vectors may contain up to 8 kb of heterologous (therapeutic) DNA in place of the viral genes. The heterologous DNA may include a tissue-specific promoter and an SDF-1 nucleic acid. In methods of delivery to an infarcted myocardium, it may also encode a ligand to a myocardial specific receptor.

Additional retroviral vectors that might be used are replication-defective lentivirus-based vectors, including human immunodeficiency (HIV)-based vectors. See, e.g., Vigna and Naldini, J. Gene Med. 5:308-316, 2000 and Miyoshi et al., J. Virol. 72:8150-8157, 1998. Lentiviral vectors are advantageous in that they are capable of infecting both actively dividing and non-dividing cells. They are also highly efficient at transducing human epithelial cells.

Lentiviral vectors for use in the disclosure may be derived from human and non-human (including SIV) lentiviruses. Preferred lentiviral vectors include nucleic acid sequences required for vector propagation as well as a tissue-specific promoter (e.g., myocardium) operably linked to a SDF-1 gene. These former may include the viral LTRs, a primer binding site, a polypurine tract, att sites, and an encapsidation site.

A lentiviral vector may be packaged into any suitable lentiviral capsid. The substitution of one particle protein with another from a different virus is referred to as "pseudotyping". The vector capsid may contain viral envelope proteins from other viruses, including murine leukemia virus (MLV) or vesicular stomatitis virus (VSV). The use of the VSV G-protein yields a high vector titer and results in greater stability of the vector virus particles.

Alphavirus-based vectors, such as those made from semliki forest virus (SFV) and sindbis virus (SIN), might also be used in the invention. Use of alphaviruses is described in Lundstrom, K., Intervirology 43:247-257, 2000 and Perri et al., Journal of Virology 74:9802-9807, 2000. Alphavirus vectors typically are constructed in a format known as a replicon. A replicon may contain (1) alphavirus genetic elements required for RNA replication, and (2) a heterologous nucleic acid such as one encoding a SDF-1 nucleic acid. Within an alphavirus replicon, the heterologous nucleic acid may be operably linked to a tissue-specific (e.g., myocardium) promoter or enhancer.

Recombinant, replication-defective alphavirus vectors are advantageous because they are capable of high-level heterologous (therapeutic) gene expression, and can infect a wide target cell range. Alphavirus replicons may be targeted to specific cell types (e.g., cardiomyocytes) by displaying on their virion surface a functional heterologous ligand or binding domain that would allow selective binding to target cells expressing a cognate binding partner. Alphavirus replicons may establish latency, and therefore long-term heterologous nucleic acid expression in a target cell. The replicons may also exhibit transient heterologous nucleic acid expression in the target cell. A preferred alphavirus vector or replicon is non-cytopathic.

In many of the viral vectors compatible with methods of the disclosure more than one promoter can be included in the vector to allow more than one heterologous gene to be expressed by the vector. Further, the vector can comprise a sequence which encodes a signal peptide or other moiety which facilitates the secretion of a SDF-1 gene product from the target cell.

To combine advantageous properties of two viral vector systems, hybrid viral vectors may be used to deliver a SDF-1 nucleic acid to a target tissue (e.g., myocardium). Standard techniques for the construction of hybrid vectors are well-known to those skilled in the art. Such techniques can be found, for example, in Sambrook, et al., In Molecular Cloning: A laboratory manual. Cold Spring Harbor, N.Y. or any number of laboratory manuals that discuss recombinant DNA technology. Double-stranded AAV genomes in adenoviral capsids containing a combination of AAV and adenoviral ITRs may be used to transduce cells. In another variation, an AAV vector may be placed into a "gutless", "helper-dependent" or "high-capacity" adenoviral vector. Adenovirus/AAV hybrid vectors are discussed in Lieber et al., J. Virol. 73:9314-9324, 1999. Retrovirus/adenovirus hybrid vectors are discussed in Zheng et al., Nature Biotechnol. 18:176-186, 2000. Retroviral genomes contained within an adenovirus may integrate within the target cell genome and effect stable SDF-1 gene expression.

Other nucleotide sequence elements which facilitate expression of the SDF-1 gene and cloning of the vector are further contemplated. For example, the presence of enhancers upstream of the promoter or terminators downstream of the coding region, for example, can facilitate expression.

In accordance with another aspect of the present disclosure, a tissue-specific promoter, such as tissue-specific transcriptional control sequences of left ventricular myosin light chain-2 (MLC₂ᵥ) or myosin heavy chain (MHC), can be fused to a SDF-1 gene. By fusing such tissue specific promoter within the adenoviral construct, transgene expression is limited to ventricular cariomyocytes. The efficacy of gene expression and degree of specificity provided by tissue specific promoters can be determined, using the recombinant adenoviral system of the present invention. Cardiac-specific (i.e., myocardial tissue specific) expression is well known in the art. (J. Biol. Chem., 267:15875-15885, 1992). Other promoters, such as the troponin-C promoter, can also be used.

The use of tissue specific promoters directed to cardiomyocytes alone (i.e., without concomitant expression in endothelial cells, smooth muscle cells, and fibroblasts within the heart) when delivering the SDF-1 gene in vivo provides adequate expression of the SDF-1 protein for therapeutic treatment. Limiting expression to the cardiomyocytes also has advantages regarding the utility of gene transfer for the treatment of CHF. In addition, cardiomyocytes would likely provide the longest transgene expression since the cells do not undergo rapid turnover; expression would not therefore be decreased by cell division and death as would occur with endothelial cells. Endothelial-specific promoters are already available for this purpose (Lee, et al., J. Biol. Chem., 265:10446-10450, 1990).

In addition to viral vector-based methods, non-viral methods may also be used to introduce a SDF-1 gene into a target cell. A review of non-viral methods of gene delivery is provided in Nishikawa and Huang, Human Gene Ther. 12:861-870, 2001. A preferred non-viral gene delivery method according to the invention employs plasmid DNA to introduce a SDF-1 nucleic acid into a cell. Plasmid-based gene delivery methods are generally known in the art.

Synthetic gene transfer molecules can be designed to form multimolecular aggregates with plasmid DNA (e.g., harboring a SDF-1 coding sequence operably linked to a myocardium-specific promoter). These aggregates can be designed to bind to a target cell (e.g., cardiomyocyte).

Cationic amphiphiles, including lipopolyamines and cationic lipids, may be used to provide receptor-independent SDF-1 nucleic acid transfer into target cells (e.g., cardiomyocytes). In addition, preformed cationic liposomes or cationic lipids may be mixed with plasmid DNA to generate cell-transfecting complexes. Methods involving cationic lipid formulations are reviewed in Felgner et al., Ann. N.Y. Acad. Sci. 772:126-139, 1995 and Lasic and Templeton, Adv. Drug Delivery Rev. 20:221-266, 1996. For gene delivery, DNA may also be coupled to an amphipathic cationic peptide (Fominaya et al., J. Gene Med. 2:455-464, 2000).

Methods that involve both viral and non-viral based components may be used according to the invention. For example, an Epstein Barr virus (EBV)-based plasmid for therapeutic gene delivery is described in Cui et al., Gene Therapy 8:1508-1513, 2001. Additionally, a method involving a DNA/ligand/polycationic adjunct coupled to an adenovirus is described in Curiel, D. T., Nat. Immun. 13:141-164, 1994.

Vectors that encode the expression of SDF-1 can be delivered to the target cell in the form of an injectable preparation containing pharmaceutically acceptable carrier, such as saline, as necessary. Other pharmaceutical carriers, formulations and dosages can also be used in accordance with the present invention.

Where the target cell comprises a cell of the infarcted myocardium, the vector can be delivered by direct intracoronary injection using a tuberculin syringe under fluoroscopic guidance, at an amount sufficient for the SDF-1 protein to be expressed to a degree which allows for highly effective therapy. By injecting the vector directly into the infarcted myocardial tissue, it is possible to target the gene rather effectively, and to minimize loss of the recombinant vectors.

This type of injection enables local transfection of a desired number of cells, especially cardiomyocytes, in the affected myocardium, thereby maximizing therapeutic efficacy of gene transfer, and minimizing the possibility of an inflammatory response to viral proteins. A cardiomyocyte-specific promoter may be used, for example, to securely enable expression limited to the cardiomyocytes. Thus, delivery of the transgenes in this matter may result in targeted gene expression in, for example, the cells of the left ventricle. Other techniques well known in the art can also be used for transplanting the vector to the target cells of the infarcted myocardium.

Where the target cell is a cultured cell that is later transplanted into the infarcted myocardium, the vectors can be delivered by direct injection into the culture medium. A SDF-1 nucleic acid transfected into cells may be operably linked to any suitable regulatory sequence, including a myocardium specific promoter and enhancer.

The transfected target cells can then be transplanted to the infarcted myocardium by well known transplantation techniques, such as by direct intracoronary injection using a tuberculin syringe. By first transfecting the target cells in vitro and then transplanting the transfected target cells to the infarcted myocardium, the possibility of inflammatory response in the infarcted myocardium is minimized compared to direct injection of the vector into the infarcted myocardium.

SDF-1 nucleic acids of the present disclosure may be expressed for any suitable length of time within the target cell, including transient expression and stable, long-term expression. The SDF-1 nucleic acid may be expressed in therapeutic amounts for a suitable and defined length of time.

A therapeutic amount is an amount, which is capable of producing a medically desirable result in a treated animal or human. As is well known in the medical arts, dosage for any one animal or human depends on many factors, including the subject's size, body surface area, age, the particular composition to be administered, sex, time and route of administration, general health, and other drugs being administered concurrently. Specific dosages of proteins, nucleic acids, or small molecules) can be determined readily determined by one skilled in the art using the experimental methods described below.

The SDF-1 expression may be transient as is the case when a cell that is not transfected with an SDF-1 protein encoding vector is transplanted into the infarcted myocardium. Alternatively, SDF-1 protein expression may be long-term, as is the case where the infarcted myocardium is transfected with an SDF-1 protein encoding vector or where a cell that is transfected with an SDF-1 protein encoding vector is transplanted to the infarcted myocardium. The latter is that aspect to which the present invention pertains.

Long term SDF-1 expression is advantageous because it allows the concentration of stem cells to be increased with a mobilizing agent, such as G-CSF or some other factor, at a time remote from the surgery or procedure that transplanted the cells. In the case where G-CSF is the mobilizing agent there is a significant increase in neutrophil count, which could cause negative effects in the peri-surgical period, but not days or weeks later. Additionally, long term or chronic up-regulation SDF-1 protein expression would allow multiple attempts at stem cell mobilization. Further, chronic up-regulation in SDF-1 protein expression causes long term homing of stem cells into the infarcted myocardial tissue from the peripheral blood without the need of stem cell mobilization.

### Stem cell mobilization

In accordance with another aspect of the present disclosure, the concentration of the stem cells in the peripheral blood of the subject can be increased by administering an agent to induce mobilization of stem cells to the peripheral blood of the subject. The stems cells can be mobilized to the peripheral blood of the subject to increase stem cell concentration in peripheral subject using a number of agents. For example, to increase the number of stem cells in the peripheral blood of a mammalian subject, an agent that causes a pluripotent stem cell to mobilize from the bone marrow can be administered to the subject. A number of such agents are known and include cytokines, such as granulocyte-colony stimulating factor (G-CSF), granulocyte-macrophage colony stimulating factor (GM-CSF), interleukin (IL)-7, IL-3, IL-12, stem cell factor (SCF), and flt-3 ligand; chemokines such as IL-8, Mip-1α, and Groβ, and the chematherapeutic agents of cylcophosamide (Cy) and paclitaxel. These agents differ in their time frame to achieve stem cell mobilization, the type of stem cell mobilized, and efficiency.

The mobilizing agent can be administered by direct injection of the mobilizing agent into the subject. Preferably, the mobilizing agent is administered after SDF-1 expression is up-regulated in the infarcted myocardium. The mobilizing agent, however, can be administered before SDF- expression is up-regulated.

A preferred mobilizing agent is a colony stimulating factor, such as G-CSF. A typical dosage of G-CSF in a murine subject is about 125 µg/Kg per day for about 5 to about 10 days. The G-CSF agent is administered after the SDF-expression is up-regulated.

Alternatively, as is well known in the art, the concentration of stem cells in the peripheral blood can be increased by the injection of specialized stem cells into the peripheral blood (i.e., MAPC's).

### VEGF

In accordance with another aspect of the present disclosure, the VEGF that is expressed in the infarcted myocardium is one of the family of vascular endothelial growth factors that induce the growth of new collateral blood vessels. VEGFs are specific angiogenic growth factors that have vaso-permeability activity and target endothelial cells almost exclusively.

The VEGF that is expressed in the infarcted myocardium can be an expression product of a VEGF gene. Preferred VEGFs that can be used in accordance with the present invention include VEGF-1 (also known as VEGF-A) and other structurally homologous VEGF's, such as VEGF-2 (VEGF-C), VEGF-3 (VEGF-B), VEGF-D, VEGF-E, and placental growth factor. Known isoforms of VEGF-1 include 121, 138, 162, 165, 182, 189, and 206 amino acids. These isoforms are identified, respectively, as VEGF-121, VEGF-165, VEGF-162, VEGF-182, VEGF-189, and VEGF-206. The mitogenic and heparin binding activity of these isoforms differ. A preferred isoform of VEGF-1 used in accordance with the present disclosure is VEGF-165. Other isoforms of VEGF-1 and other homologs of VEGF not listed can also be used in accordance with the present disclosure.

The VEGF of the present disclosure can have an amino acid sequence identical to one of the foregoing VEGFs. The VEGF of the present disclosure can also be a variant of one of the foregoing VEGFs, such as a fragment, analog and derivative of VEGF. Such variants include, e.g., a polypeptide encoded by a naturally occurring allelic variant of native VEGF gene (i.e., a naturally occurring nucleic acid that encodes a naturally occurring mammalian VEGF), a polypeptide encoded by an alternative splice form of a native VEGF gene, a polypeptide encoded by a homolog of a native VEGF gene, and a polypeptide encoded by a non-naturally occurring variant of a VEGF gene.

VEGF variants have a peptide sequence that differs from a native VEGF in one or more amino acids. The peptide sequence of such variants can feature a deletion, addition, or substitution of one or more amino acids of a native VEGF. Amino acid insertions are preferably of about 1 to 4 contiguous amino acids, and deletions are preferably of about 1 to 10 contiguous amino acids. Variant VEGF in accordance with the present disclosure substantially maintain a native VEGF functional activity. Preferred VEGF protein variants can be made by expressing nucleic acid molecules within the disclosure that feature silent or conservative changes.

VEGF fragments corresponding to one or more particular motifs and/or domains or to arbitrary sizes, are within the scope of the present disclosure Isolated peptidyl portions of VEGF can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry.

Variants of VEGF can also include recombinant forms of the VEGF. Recombinant polypeptides preferred by the present disclosure, in addition to a VEGF protein, are encoded by a nucleic acid that has at least 85% sequence identity with the nucleic acid sequence of a gene encoding a mammalian VEGF.

VEGF variants can include agonistic forms of the protein that constitutively express the functional activities of a native VEGF. Other VEGF variants can include those that are resistant to proteolytic cleavage, as for example, due to mutations which alter protease target sequences. Whether a change in the amino acid sequence of a peptide results in a variant having one or more functional activities of a VEGF can be readily determined by testing the variant for VEGF functional activity.

### VEGF Nucleic Acids

Another aspect of the present disclosure relates to nucleic acid molecules that encode VEGF and non-native nucleic acids that encode a mammalian VEGF. Such nucleic acid molecules may be in the form of RNA or in the form of DNA (e.g., cDNA, genomic DNA, and synthetic DNA). The DNA may be double-stranded or single-stranded, and if single-stranded may be the coding (sense) strand or non-coding (anti-sense) strand.

Other nucleic acid molecules within the disclosure are variants of a native VEGF gene, such as those that encode fragments, analogs and derivatives of a native VEGF. Such variants may be, e.g., a naturally occurring allelic variant of a VEGF gene, a homolog of a native VEGF gene, or a non-naturally occurring variant of a native VEGF gene. These variants have a nucleotide sequence that differs from a native VEGF gene in one or more bases. For example, the nucleotide sequence of such variants can feature a deletion, addition, or substitution of one or more nucleotides of a native VEGF gene. Nucleic acid insertions are preferably of about 1 to 10 contiguous nucleotides, and deletions are preferably of about 1 to 30 contiguous nucleotides.

In other applications, variant VEGF displaying substantial changes in structure can be generated by making nucleotide substitutions that cause less than conservative changes in the encoded polypeptide. Examples of such nucleotide substitutions are those that cause changes in (a) the structure of the polypeptide backbone; (b) the charge or hydrophobicity of the polypeptide; or (c) the bulk of an amino acid side chain. Nucleotide substitutions generally expected to produce the greatest changes in protein properties are those that cause non-conservative changes in codons. Examples of codon changes that are likely to cause major changes in protein structure are those that cause substitution of (a) a hydrophilic residue, e.g., serine or threonine, for (or by) a hydrophobic residue, e.g., leucine, isoleucine, phenylalanine, valine or alanine; (b) a cysteine or proline for (or by) any other residue; (c) a residue having an electropositive side chain, e.g., lysine, arginine, or histidine, for (or by) an electronegative residue, e.g., glutamine or aspartine; or (d) a residue having a bulky side chain, e.g., phenylalanine, for (or by) one not having a side chain, e.g., glycine.

Naturally occurring allelic variants of a VEGF gene within the disclosure are nucleic acids isolated from mammalian tissue that have at least 75% sequence identity with a native VEGF gene, and encode polypeptides having structural similarity to a native VEGF. Homologs of a native VEGF within the disclosure are nucleic acids isolated from other species that have at least 75% sequence identity with the native gene, and encode polypeptides having structural similarity to a native VEGF. Public and/or proprietary nucleic acid databases can be searched to identify other nucleic acid molecules having a high percent sequence identity to a native VEGF gene.

Non-naturally occurring VEGF variants are nucleic acids that do not occur in nature (e.g., are made by the hand of man), have at least 75% sequence identity with a native VEGF gene, and encode polypeptides having structural similarity to a native VEGF. Examples of non-naturally occurring VEGF gene variants are those that encode a fragment of a native VEGF, those that hybridize to a native VEGF gene or a complement of to a native VEGF gene under stringent conditions, those that share at least 65% sequence identity with a native VEGF gene or a complement of a native VEGF gene, and those that encode a VEGF.

Nucleic acids encoding fragments of a VEGF within the disclosure are those that encode, amino acid residues of a native VEGF. Shorter oligonucleotides that encode or hybridize with nucleic acids that encode fragments of a native VEGF can be used as probes, primers, or antisense molecules. Longer polynucleotides that encode or hybridize with nucleic acids that encode fragments of a native VEGF can also be used in various aspects of the disclosure. Nucleic acids encoding fragments of a native VEGF can be made by enzymatic digestion (e.g., using a restriction enzyme) or chemical degradation of the full length native VEGF gene or variants thereof.

Nucleic acids that hybridize under stringent conditions to one of the foregoing nucleic acids can also be used in the disclosure. For example, such nucleic acids can be those that hybridize to one of the foregoing nucleic acids under low stringency conditions, moderate stringency conditions, or high stringency conditions are within the disclosure.

Nucleic acid molecules encoding a VEGF fusion protein may also be used in the disclosure. Such nucleic acids can be made by preparing a construct (e.g., an expression vector) that expresses a VEGF fusion protein when introduced into a suitable target cell. For example, such a construct can be made by ligating a first polynucleotide encoding VEGF fused in frame with a second polynucleotide encoding another protein such that expression of the construct in a suitable expression system yields a fusion protein.

The oligonucleotides of the disclosure can be DNA or RNA or chimeric mixtures or derivatives or modified versions thereof, single-stranded or double-stranded. Such oligonucleotides can be modified at the base moiety, sugar moiety, or phosphate backbone, for example, to improve stability of the molecule, hybridization, etc. Oligonucleotides within the invention may additionally include other appended groups such as peptides (e.g., for targeting cell receptors in vivo), or agents facilitating transport across the cell membrane. To this end, the oligonucleotides may be conjugated to another molecule, e.g., a peptide, hybridization triggered cross-linking agent, transport agent, hybridization-triggered cleavage agent, etc.

### VEGF Expression

In accordance with another aspect of the present disclosure, the expression VEGF in the infarcted myocardium can be increased by introducing an agent into target cells that increases expression of VEGF.

The target cells can include cells within the infarcted myocardium or ex vivo cells, such as autologous skeletal myoblasts or fibroblasts, which are transplanted into the infarcted myocardial tissue following introduction of the agent. Where the target cells are cells that are transplanted into the infarcted myocardium, the target cell can be same cells as the cells used to promote up-regulation of SDF-1 protein or transfected with a SDF-1 protein encoding vector.

The agent can comprise natural or synthetic VEGF nucleic acids that are incorporated into recombinant nucleic acid constructs, typically DNA constructs, capable of introduction into and replication in the cell. Such a construct preferably includes a replication system and sequences that are capable of transcription and translation of a polypeptide-encoding sequence in a given target cell.

Other agents can also be introduced the target cells to increase VEGF levels in a target tissue. For example, agents that increase the transcription of a gene encoding VEGF; increase the translation of an mRNA encoding VEGF, and/ or those that decrease the degradation of an mRNA encoding VEGF could be used to increase VEGF levels. Increasing the rate of transcription from a gene within a cell can be accomplished by introducing an exogenous promoter upstream of the gene encoding VEGF. Enhancer elements which facilitate expression of a heterologous gene may also be employed.

A preferred method of introducing the agent into a target cell involves using gene therapy. Gene therapy in accordance with the present disclosure can be used to express VEGF from a target cell in vivo or in vitro.

A preferred gene therapy method involves using a vector including a nucleotide encoding VEGF. Examples of vectors that can be used include, for example, viral vectors (such as adenoviruses ('Ad'), adeno-associated viruses (AAV); and retroviruses), liposomes and other lipid-containing complexes, and other macromolecular complexes capable of mediating delivery of a polynucleotide to a target cell.

The vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities.

Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (i.e. positive/negative) markers. Such marker genes can provide an added measure of control that can be advantageous in gene therapy contexts. A large variety of such vectors are known in the art and are generally available.

Vectors for use in expressing VEGF in the present invention include viral vectors, lipid based vectors and other vectors that are capable of delivering nucleotide according to the present disclosure to the target cells. The vector can be a targeted vector, especially a targeted vector that preferentially binds to ventricular myocytes. Preferred viral vectors for use in the disclosure are those that exhibit low toxicity to a target cell and induce production of therapeutically useful quantities of VEGF in a tissue-specific manner.

Presently preferred viral vectors are derived from adenovirus (Ad) or adeno-associated virus (AAV). Both human and non-human viral vectors can be used but preferably the recombinant viral vector is replication-defective in humans. Where the vector is an adenovirus, it preferably comprises a polynucleotide having a promoter operably linked to a gene encoding an angiogenic protein or peptide, and is replication-defective in humans. Other vectors including viral and non-viral vectors well known in the art and described above can also be used.

In many of the viral vectors compatible with methods of the disclosure, more than one promoter can be included in the vector to allow more than one heterologous gene to be expressed by the vector. Further, the vector can comprise a sequence which encodes a signal peptide or other moiety which facilitates the secretion of a VEGF product from the target cell.

To combine advantageous properties of two viral vector systems, hybrid viral vectors may be used to deliver a VEGF nucleic acid to a target tissue (e.g., myocardium). Other nucleotide sequence elements which facilitate expression of the VEGF gene and cloning of the vector are further contemplated. For example, the presence of enhancers upstream of the promoter or terminators downstream of the coding region, for example, can facilitate expression. In the vectors of the present disclosure, the presence of elements which enhance myocardium-specific expression of VEGF may be useful for gene therapy.

The present disclosure also contemplates the use of tissue-specific promoters for cell targeting. By fusing, for example, tissue-specific transcriptional control sequences of left ventricular myosin light chain-2 (MLC₂ᵥ) or myosin heavy chain (MHC) to a transgene, such as the VEGF-165 gene within the adenoviral construct, transgene expression is limited to ventricular cardiac myocytes.

By using the MLC₂ᵥ or MHC promoters and delivering the transgene in vivo, it is believed that the cardiomyocyte alone (that is without concomitant expression in endothelial cells, smooth muscle cells, and fibroblasts within the heart) will provide adequate expression of an angiogenic protein, such as VEGF-165 to promote angiogenesis. Limiting expression to cardiomyocytes also has advantages regarding the utility of gene transfer for treatment of congestive heart failure. By limiting expression to the heart, one avoids the potentially harmful effect of angiogenesis in non-cardiac tissues. In addition, of the cells in the heart, the myocyte would likely provide the longest transgene expression since the cells do not undergo rapid turnover; expression would not therefore be decreased by cell division and death as would occur with endothelial cells.
Endothelial-specific promoters are already available for this purpose.

By using drug-regulatable promoter systems (e.g., tetracycline), SDF-1 expression with or without stem cell mobilization can be started at times remote from surgical or percutaneous procedures necessary for implantation of engineered cells or genetic vectors. This would allow recovery of myocardial or non-myocardial tissues prior to increasing the number of circulating stem cells.

In addition to viral vector-based methods, non-viral methods may also be used to introduce a VEGF gene into a target cell. A preferred non-viral gene delivery method according to the disclosure employs plasmid DNA to introduce a VEGF nucleic acid into a cell. Plasmid-based gene delivery methods are generally known in the art.

Methods that involve both viral and non-viral based components may be used according to the disclosure. For example, an Epstein Barr virus (EBV)-based plasmid for therapeutic gene delivery is described in Cui et al., Gene Therapy 8:1508-1513, 2001. Additionally, a method involving a DNA/ligand/polycationic adjunct coupled to an adenovirus is described in Curiel, D. T., Nat. Immun. 13:141-164, 1994.

The vectors that encode the expression of VEGF can be delivered to the target cell in the form of an injectable preparation containing pharmaceutically acceptable carrier such as saline, for example, as necessary. Other pharmaceutical carriers, formulations and dosages can also be used.

Where the target cell comprises a cell of the infarcted myocardium, the vector can be delivered by direct intracoronary (or graft vessel) injection using a tuberculin syringe under fluoroscopic guidance, at an amount sufficient for the VEGF to be expressed to a degree which allows for highly effective therapy. By injecting the vector directly into the infarcted myocardial tissue, it is possible to target the gene rather effectively, and to minimize loss of the recombinant vectors.

This type of injection enables local transfection of a desired number of cells, especially cardiac myocytes, in the affected myocardium, thereby maximizing therapeutic efficacy of gene transfer, and minimizing the possibility of an inflammatory response to viral proteins. A ventricular cardiomyocyte-specific promoter may be used, for example, to securely enable expression limited to the cardiomyocytes. Thus, delivery of the transgenes in this matter may result in targeted gene expression in, for example, the cells of the left ventricle. Other techniques well known in the art can also be used for transplanting the vector to the target cells of the infarcted myocardium.

Where the target cell is a cultured cell that is later transplanted into the infarcted myocardium, the vectors can be delivered by direct injection into the culture medium. A VEGF nucleic acid transfected into cells may be operably linked to any suitable regulatory sequence, including a myocardium specific promoter and enhancer.

The transfected target cells can then be transplanted to the infarcted myocardium by well known transplantation techniques, such as by direct intracoronary injection using a tuberculin syringe. By first transfecting the target cells in vitro and than transplanting the transfected target cells to the infarcted myocardium, the possibility of inflammatory response in the infarcted myocardium is minimized compared to direct injection of the vector into the infarcted myocardium.

VEGF of the present disclosure may be expressed for any suitable length of time including transient expression and stable, long-term expression. In a preferred embodiment, the SDF-1 nucleic acid will be expressed in therapeutic amounts for a suitable and defined length of time.

### Examples

The present disclosure is further illustrated by the following specific examples. The invention to which the disclosure pertains is illustrated by the example labelled "3" on pages 68-70.

### 1. Effect of stem cell mobilization with G-CSF 8 weeks following MI

In order to ascertain whether stem cell mobilization by G-CSF leads to myocardial regeneration in rats with established ischemic cardiomyopathy, rats 8 weeks following MI were randomized to receive either recombinant human G-CSF (125 µg/kg/day for 5 days, via i.p. injection) or saline. Blood was obtained 5 days after initiating G-CSF therapy to verify bone marrow stimulation, revealing a tripling of the leukocyte count with G-CSF (37.3 + 5.3 cells/µl) compared with saline (11.8 + 4.0 cells/µl) therapy. 5-bromo- 2'-deoxyuridine, BrdU, was administered beginning on the final day after G-CSF administration for a total of 14 days in order to label any proliferating cells with in the myocardium 20.

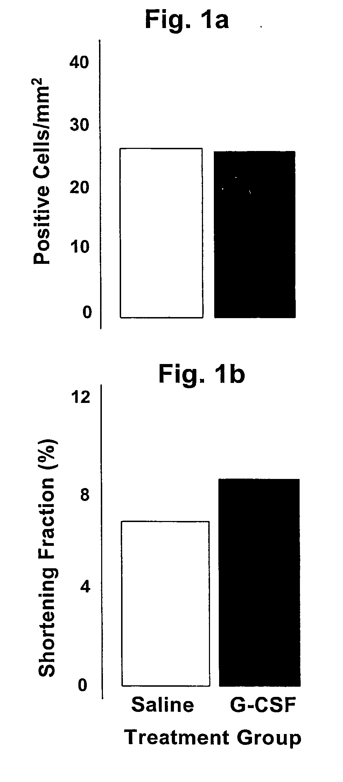

Figs. 1(a and b) show, respectively, the number BrdU-positive cells within infarct zone (a) and the shortening fraction (b) 4 weeks following administration of saline or G-CSF (12 weeks following LAD ligation). Cell counts are cells per mm². Data represent mean ± s.d. *n*=6-8 per group.

The administration of G-CSF 2 months after LAD ligation did not lead to an increase in BrdU positive cell number within the infarct zone (Fig. 1a) or to meaningful myocardial regeneration as determined by the lack of angiogenesis or cardiac myosin positive cells within the infarct zone (data not shown). Twelve weeks following LAD ligation these animals demonstrated a significant cardiomyopathy with a shortening fraction in control animals of significant less than 10% (normal >60%). Consistent with lack of histological evidence of significant myocardial regeneration in response to G-CSF 8 weeks after LAD ligation, no meaningful recovery of systolic function was seen with G-CSF (Fig. 1b).

### 2. Effect of SKMB transplantation prior to stem cell mobilization on ischemic cardiomyopathy

In order to test the hypothesis that myocardium temporally remote from the time of myocardial infarction can be optimized for myocardial regeneration in response to stem cell mobilization, skeletal myoblast transplantation was performed 8 weeks following LAD ligation. Animals received 5 injections of 200,000 SKMB/injection within the infarct border zone. Because the initial hypothesis was that the transplanted SKMB would be used as a strategy for expressing gene products responsible for stem'cell homing, as a control, the SKMB were transfected with adenovirus encoding luciferase.

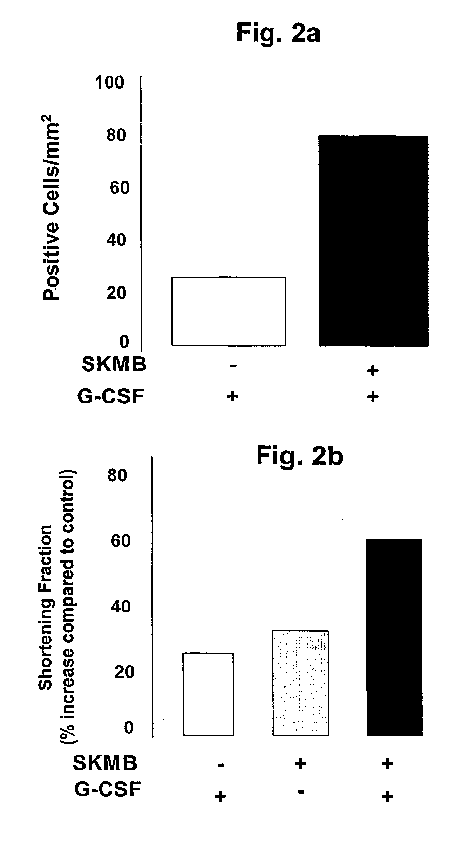

Figs. 2(a and b) show the effect of skeletal myoblast (SKMB) transplantation on BrdU+ cell counts within the infarct zone 4 weeks following cell transplantation (12 weeks following LAD ligation). Data represent mean ± s.d. *n*=6-8 per group.

The introduction of SKMB into the infarcted heart in the absence of G-CSF did not significantly increase the incorporation of BrdU positive cells into the infarct zone. However, the combination of SKMB transplantation and G-CSF did result in a significantly increased number of BrdU positive cells within the infarct zone 4 weeks later (Fig. 2a). In addition, compared to animals that received either G-CSF or SKMB transplantation alone, animals that received the combined therapy experienced a significant increase in shortening fraction (Fig. 2b) relative to saline controls.

In order to determine if the BrdU positive cells in the infarct zone originated in the bone marrow, or were endogenous cells from the myocardium that divided, 8 weeks following LAD ligation, BrdU was administered, for 5 days, starting 6 days prior to transplantation of SKMB and initiation of G-CSF.

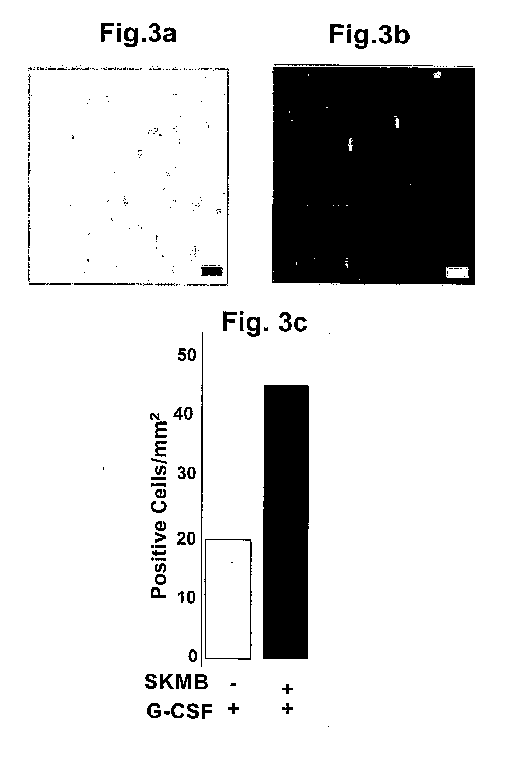

Fig. 3a shows that bone marrow (BM) stained for BrdU revealed almost 100% staining of BM cells cultured from multiple animals. Fig. 3b shows that no significant Brdu+ cells were seen in untreated myocardium after 5 days of BrdU administration. Data represent mean ± s.d. of positive cells quantified by two independent observers blinded to the identity of each animal. *n*=6-8 per group. Scale bar represents 25 µM. This led to labeling of cells in the bone marrow without any significant Brdu labeling of the myocardium (17.5 ± 2.9 positive cells/mm²).

Fig. 3c shows the increased BrdU+ cells within the infarct zone assessed with the therapy in accordance with the present invention.

In these experiments, only those animals that received SKMB and G-CSF had a significant increase in the number of BrdU-positive cells within the infarct zone. These data are consistent with the concept that the BrdU-positive cells arose from the bone marrow and homed to the infarct zone following combined therapy. The data also support that SKMB transplantation re-establishes the necessary signals for stem cell homing to the myocardium.

### 3. Signaling molecules responsible for stem cell homing to infarcted tissue

The observation that circulating cells will engraft into infarcted tissue 8 weeks after an MI with "stimulation" of the tissue by SKMB transplantation prompted the evaluation of potential mediators of stem cell homing. Stromal cell-derived factor-1 (SDF-1) is known to mediate hematopoietic trafficking and stem cell homing in the bone marrow; therefore, its role as a potential signaling molecule for stem cell engraftment in MI and in response to SKMB transplantation was assessed.

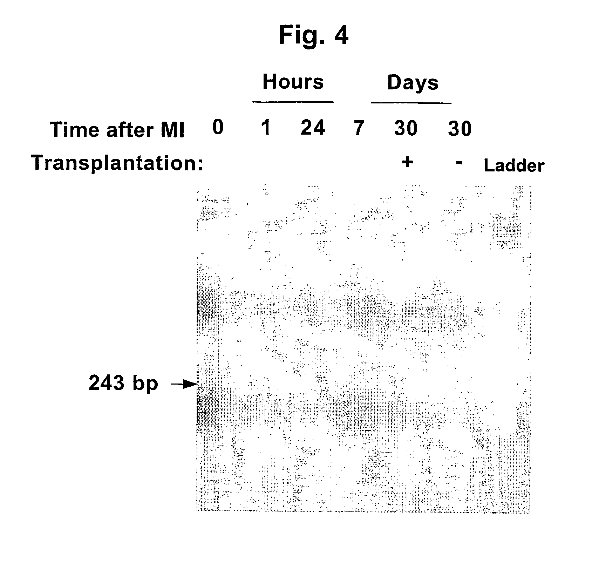

Fig. 4 is a photograph showing RT-PCR revealing stromal derived factor-1 (SDF-1) expression as a function of time following myocardial infarction. SDF-1 expression is absent at baseline, increased at 1 and 24 hours following MI. SDF-1 expression returns to its absent state between 24 hours and 7 days following MI and remains absent 30 days following MI. SDF-1 expression recurs 72 hours following SKMB transplantation performed 30 days (30+) following MI.

Thus, by RT-PCR, SDF-1 expression was observed at 1 and 24 h, but not at 0, 7 or 30 days, after LAD ligation. SDF-1 expression was induced by SKMB, and was observed 72 h after transplantation, but not in sham operated animals (data not shown). PCR for GAPDH in the same samples demonstrated that cDNA was intact in all samples (data not shown). The increase in SDF-1 expression in response to SKMB transplantation was confirmed by real-time PCR (data not shown). Real-time PCR revealed GAPDH levels were similar among groups.

To evaluate whether SDF-1 mediated engraftment of BrdU positive cells into the infarct zone, control cardiac fibroblasts or those stably transfected with an SDF-1 expression vector were transplanted into myocardium 4 weeks following LAD ligation. Ten days later, to allow for down-regulation of endogenous SDF-1 expression, G-CSF was administered for 5 days, as was BrdU for 5 days beginning on the final day of G-CSF administration.

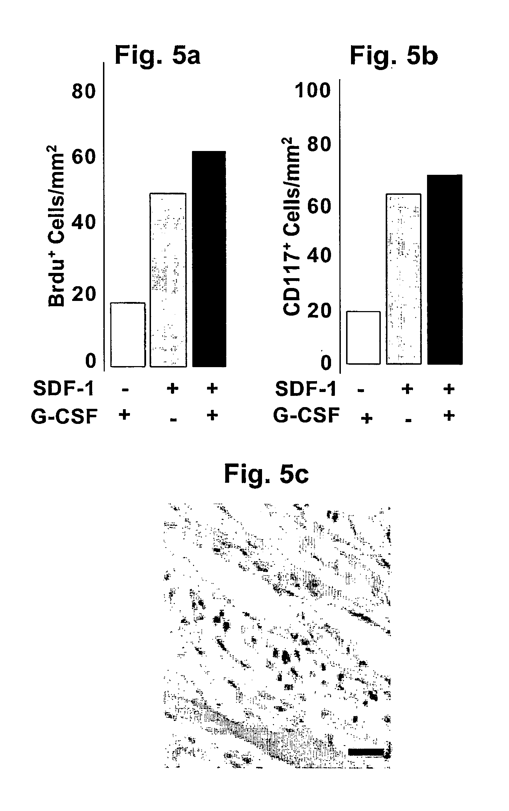

Fig. 5(a and b) show the number of (a) BrdU+ cells and (b) CD117+ cells within the infarct zone 4 weeks following transplantation of cardiac fibroblasts stably transfected with or without SDF-1 expression vector with or without G-CSF administration for 5 days following cardiac fibroblast transplantation. Data represent mean ± s.d. of positive cells quantified by two independent observers blinded to the identity of each animal. *n*=3-5 per group.

Fig. 5c is a photograph from a SDF-1/G-CSF treated animal stained CD117+. Scale bar represents 25 µM.

Consistent with SDF-1 being sufficient to induce stem cell homing to injured myocardium, in response to G-CSF, hearts transplanted with SDF-1-expressing cardiac fibroblasts revealed a greater than 3-fold increase in the number of BrdU-positive cells throughout the infarct zone. Animals that received control cardiac fibroblasts had a BrdU signal that was no different from control.

### 4. Identification of BrdU positive cells

performed immunofluorescence in order to determine the identity of the BrdU cells within the infarct zone. Antibody staining for CD45 demonstrated that <5% of the BrdU-positive cells in response to cell transplantation and G-CSF administration are leukocytes, respectively. No cardiac myosin-BrdU positive cells were observed within the infarct zone of the animals treated with G-CSF without or with SKMB or cardiac fibroblast transplantation.

### 5.Effect of VEGF expressing SKMB and G-CSF on neovascularization and LV function late following MI

Despite an increased number of BrdU-positive cells in the animals that received combined SKMB transplantation and bone marrow stimulation with G-CSF, an increase in the vascular density or increase in the number of cardiac myocytes within the infarct zone was not observed. Therefore, we studied the added effect of VEGF over-expression on SKMB transplantation and G-CSF administration.

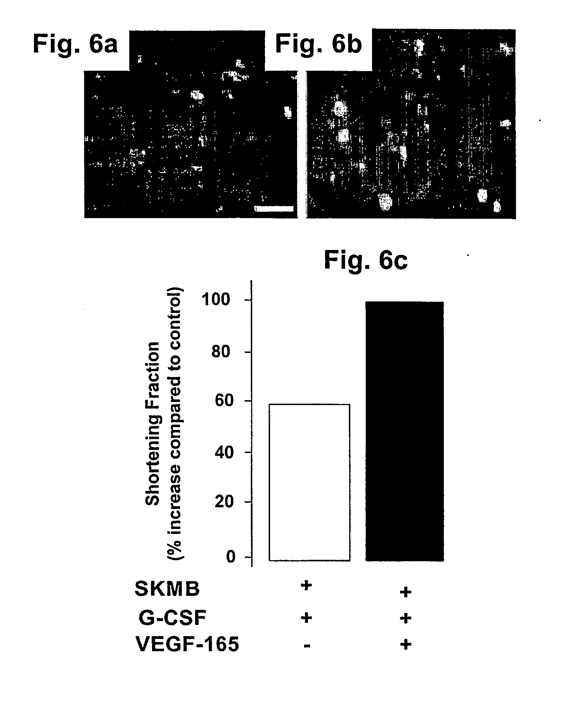

Figs. 6(a and b) show that the immunohistochemistry of the infarct zone revealed both BrdU + cells (open arrows) and cardiac myosin-expressing cells (closed arrows) 12 weeks following LAD ligation with cell transplantation of (a) SKMB or (b) VEGF-expressing SKMB followed by stem cell mobilization using G-CSF.

Fig. 6c shows improvement in LV function relative to no treatment control. Data represent mean ± s.d. n=6-8 per group. Scale bar represents 10 µM.

Transplantation of VEGF-165-expressing SKMB 8 weeks following MI resulted in an increased vascular density within the infarct zone compared to saline controls (44.1 ± 5.2 vs. 17.7 ± 2.8 vessels/mm²; VEGF-165 vs. saline, respectively). Furthermore, the combination VEGF-165 expression and stem cell mobilization with G-CSF led to repopulation of the infarct zone with cardiac myosin-expressing cells consistent with myocardial regeneration (Fig 6a). The addition of VEGF-165 expression to SKMB also significantly increased LV function as measured by shortening fraction (Fig. 6b). No significant difference was observed in shortening fraction between treatment strategies of transplantation of VEGF-165 expressing SKMB and SKMB transplantation combined with the administration of G-CSF (data not shown).

### Methods

### LAD Ligation

All animal protocols were approved by the Animal Research Committee, and all animals were housed in the AAALAC animal facility of the Cleveland Clinic Foundation. Animals were anesthetized with sodium pentobarbital, 50 mg/kg, intubated, and ventilated with room air at 80 breaths per minute using a pressure cycled rodent ventilator (Kent Scientific Corp, RSP1002). Anterior wall MI was induced in 150-175g male Lewis rats by ligation of the left anterior descending (LAD) artery with the aid of a surgical microscope (Leica M500){}.

### 2D-Echocardiography

2D-echocardiography was performed 5-7 days and 8 weeks following LAD ligation and 4 weeks following SKMB transplantation using a 15-MHz linear array transducer interfaced with a Sequoia C256 (Acuson). Rats were lightly sedated with ketamine (50mg/kg) for each echocardiogram. For quantification of LV dimensions and wall thickness, we digitally recorded 2D clips and m-mode images in a short axis view from the mid-LV just below the papillary muscles allowing for consistent measurements from the same anatomical location in different rats. Measurements were made by two independent blinded observers offline using ProSolve. Each measurement in each animal is made 6 times, from 3 randomly chosen m-mode clips out of 5 recorded by an observer blinded to treatment arm. As a measure of LV function, the shortening fraction was calculated from M-Mode recordings. Shortening fraction (%) = (LVEDD-LVESD)/LVEDD*100, where LVEDD - left ventricular end diastolic dimension and LVESD - left ventricular end systolic dimension. Dimensions were measured between the anterior wall and posterior wall from the short axis view just below the level of the papillary muscle. In addition, anterior wall thickness was measured at end-diastole.

### Cell Preparation and Delivery

Skeletal myoblasts were harvested from the hind limbs of several Lewis rats (Harlan Labs), plated in 175ml culture flask (Falcon), and grown in DMEM including 10% fetal bovine serum, 300mg/l ECGS, and the antibiotics penicillin, streptomycin, and ofloxacin. Cells underwent passaging once 75% confluence was achieved to avoid differentiation. On the day prior to cell transplantation, purified myoblasts were transfected with 108 pfu/ml of replicative deficient adenovirus expressing VEGF-165 or luciferase (control) under control of a CMV promoter. On the day of transplantation, myoblasts were harvested with trypsin, washed extensively in PBS to remove any free viral particles and reconstituted immediately prior to transplantation. Animals were then anesthetized, ventilated and subjected to a lateral thoracotomy for direct visualization of the infarct zone. Approximately 1 x 10(6) cells were injected per animal in 5 locations.

Cardiac fibroblasts were harvested from several adult rat hearts and plated in a similar fashion to SKMB. SDF-1 from the total RNA retrieved from hearts 24 h after myocardial infarction was cloned into the expression vector PCDNA3.1 
(Forward-(NOT-1)-AATAAGAAATGCGGCCGCATGGACGCCAAGGTCGTCGC

TGTGCTGGCC;
 Reverse-(Xba-1)-TCTAGACTTGTTTAAGGCTTTGTCCAGGTACTCTTGGA.
Cardiac fibroblasts stably transfected with SDF-1 PCDNA3.1 expression vector were selected with neomycin.

### Stem Cell Mobilization

Recombinant human G-CSF (125 ug/kg) was administered via intraperitoneal (i.p.) injection for 5 days beginning on the day of skeletal myoblast transplantation. Complete blood count and differential data (Bayer, ADVIA) were obtained on day 0, 5, 14, and 21 post transplantation. In order to measure the cumulative extent of cell proliferation, 50 mg/kg of BrdU was injected i.p. for 14 days beginning on day 5 to allow for BrdU labeling of any proliferating stem cells induced by G-CSF.

### Histologic Analysis

Rats were euthanized, their hearts harvested for analysis 4 weeks following cell transplantation following perfusion fixation with HistoChoice (Amresco Inc., Solon, OH), and sectioned into 3 equal division perpendicular to the LV long-axis. The mid-ventricular and apical segments were paraffin-embedded and several sections 6 um thick were utilized for immunohistochemistry. Monoclonal antibodies to cardiac myosin (Chemicon), CD45 (Chemicon), Factor VIII (Chemicon), CD117 (Santa Cruz Biotehnology) and BrdU (Vector labs) were utilized. Secondary antibodies either FITC- or biotin-labeled were used. For quantification, five sections within the infarct zone were analyzed for positive cells and vascular density. We were unable to perform CD117 and BrdU double labeling because the HCl treatment in our protocol for BrdU antigen presentation resulted in the expression of a nuclear epitope that three different CD117 antibodies recognized.

### PCR Analysis

RT-PCR analysis was performed on total RNA isolated from rat hearts as a function of time after LAD ligation and after SKMB transplantation. Total RNA was extracted from tissue by the guanidine isothiocyanate-cesium chloride method. Primers specific for rat SDF-1 (Forward: TTGCCAGCACAAAGACACTCC; Reverse: CTCCAAAGCAAACCGAA TACAG, expected product 243 base pairs, 40 cycles) were utilized and GAPDH
(Forward: CCCCTGGCCAAGGTCATCCA; Reverse: CGGAAGGCCATGCCAGTGAG, expected product 238 base pairs, 20 cycles). Real time PCR (Perkin-Elmer, ABI Prism 7700) was then used to confirm the increase in expression within infarcted and transplanted hearts using SYBR-green incorporation into the PCR product SDF-1 (Forward: ATGCCCCTGCCGATTCTTTG Reverse: TGTTGTTGCTTTTCAGCCTTGC, expected product 116 base pairs) and GAPDH as above.

### Adenoviral Construct

The adenoviral construct encoding VEGF-165 was generous gift from Gen Vec, Inc (Gaithersburg, MD). Briefly, 293 cells were obtained from American Type Culture Collection (ATCC CRL. 1573) and were maintained in Dulbecco's modified Eagle's medium (DMEM) supplemented with 10% calf serum. The E1-,E3-adenovirus vector AdVEGF-165 was generated by linearizing the shuttle vector plasmid at a unique restriction site adjacent to the left end inverted terminal repeat (ITR) and cotransfected into 293 cells with ClaI digested H5dl324 DNA. After two sequential plaque purifications vector stocks were propagated on 293 cells and purified through three sequential bandings on cesium chloride gradients. The purified virus was dialyzed against a buffer containing 10 mM Tris, pH 7.8, 150 mM NaCl, 10 mM MgCl2 and 3% sucrose and stored at -80 0C until use. The transgene expression is under the control of the cytomegalovirus immediate early promoter.

### Statistical Analysis

Data are presented as mean ± SEM. Comparisons between groups were made by Student t-test.

## Claims

1. Use of a cell transfected with an expression vector encoding SDF-I protein to express SDF-1 protein, for the manufacture of a medicament for treating infarcted myocardial tissue in a subject.

2. A cell transfected with an expression vector encoding SDF-1 protein to express SDF-1 protein for use in treating infarcted myocardial tissue in a subject.

3. The use of claim 1 or cell for use of claim 2, wherein the cells transfected with the expression vector encoding SDF-1 protein are cardiofibroblasts.

4. The use or cell for use of claim 3, the cardiofibroblasts being genetically modified with an expression vector, the expression vector including a nucleic acid encoding for SDF-1 protein.

5. The use or cell for use of claim 3, the cardiofibroblasts being cultured *ex vivo.*

6. The use or cell for use of claim 5, the cardiofibroblasts being introduced into infarcted myocardial tissue by transplanting the cardiofibroblasts into the infarcted myocardial tissue.

## Patentansprüche

1. Verwendung einer Zelle, die mit einem Expressionsvektor transfiziert wurde, der ein SDF-1 Protein zum Exprimieren eines SDF-1 Proteins kodiert, zur Herstellung eines Medikaments zur Behandlung von infarktiertem Myokardgewebe bei einem Subjekt.

2. Zelle, die mit einem Expressionsvektor transfiziert wurde, der ein SDF-1 Protein zum Exprimieren eines SDF-1 Proteins kodiert, zur Behandlung von infarktiertem Myokardgewebe bei einem Subjekt.

3. Verwendung nach Anspruch 1 oder Zelle zur Verwendung nach Anspruch 2, wobei die Zellen, die mit dem Expressionsvektor transfiziert wurden, der ein SDF-1 Protein kodiert, Kardiofibroblasten sind.

4. Verwendung oder Zelle zur Verwendung nach Anspruch 3, wobei die Kardiofibroblasten genetisch mit einem Expressionsvektor modifiziert werden, wobei der Expressionsvektor eine Nukleinsäure zum Kodieren des SDF-1 Proteins umfasst.

5. Verwendung oder Zelle zur Verwendung nach Anspruch 3, wobei die Fibroblasten *ex vivo* gezüchtet werden.

6. Verwendung oder Zelle zur Verwendung nach Anspruch 5, wobei die Kardiofibroblasten in das infarktierte Myokardgewebe durch Transplantieren der Kardiofibroblasten in das infarktierte Myokardgewebe eingeführt werden.

## Revendications

1. Utilisation d'une cellule transfectée avec un vecteur d'expression codant une protéine SDF-1 pour exprimer une protéine SDF-1, en vue de la fabrication d'un médicament destiné au traitement du tissu myocardique infarci chez un sujet.

2. Cellule transfectée avec un vecteur d'expression codant une protéine SDF-1 pour exprimer une protéine SDF-1, en vue d'une utilisation dans le traitement du tissu myocardique infarci chez un sujet.

3. Utilisation selon la revendication 1 ou cellule en vue d'une utilisation selon la revendication 2, dans laquelle les cellules transfectées avec le vecteur d'expression codant une protéine SDF-1 sont des cardiofibroblastes.

4. Utilisation ou cellule en vue d'une utilisation selon la revendication 3, les cardiofibroblastes étant génétiquement modifiés avec un vecteur d'expression, le vecteur d'expression incluant un acide nucléique codant la protéine SDF-1.

5. Utilisation ou cellule en vue d'une utilisation selon la revendication 3, les cardiofibroblastes étant cultivés *ex vivo.*

6. Utilisation ou cellule en vue d'une utilisation selon la revendication 5, les cardiofibroblastes étant introduits dans le tissu myocardique infarci en transplantant les cardiofibroblastes dans le tissu myocardique infarci.
